# EUROPEAN PATENT SPECIFICATION

(11) **EP 0 510 151 B1**
(45) Date of publication and mention of the grant of the patent: **05.04.1995**
(21) Application number: 91919554.5
(22) Date of filing: 26.09.1991
(51) Int. Cl.: A61K 7/22, A61K 7/16

(54) **IMPROVED ANTI-PLAQUE COMPOSITIONS COMPRISING A COMBINATION OF MORPHOLINOAMINO ALCOHOL AND ANTI-MICROBIAL AGENT**
GEGEN ZAHNBELAG VERBESSERTE MITTEL ENTHALTEND EINE KOMBINATION AUS EINEM MORPHOLINOAMINO-ALKOHOL UND EIN ANTIMIKROBIELLES MITTEL
COMPOSITIONS ANTIPLAQUE AMELIOREES COMPRENANT UNE COMBINAISON D'ALCOOL MORPHOLINOAMINO ET UN AGENT ANTIMICROBIEN

(30) Priority: 09.11.1990 US 612034
(43) Date of publication of application: 28.10.1992
(73) Proprietor: Pharmacia AB, 171 97 Stockholm (SE)
(72) Inventor: DILLS, Steven, S., Hackettstown, NJ 07840 (US); LYNCH, Donald, M., Flemington, NJ 08822 (US); PAN, Pauline, H., Morris Plains, NJ 07950 (US); SHAW, Allan, Convent Station, NJ 07961 (US); STURDIVANT, Linda, D., East Orange, NJ 07017 (US)
(74) Representative: Widén, Björn
(86) International application number: US9107083
(87) International publication number: WO9208442

(56) References cited:
- EP-A- 0 373 758
- US-A- 4 636 382
- US-A- 4 894 221

## Description

### BACKGROUND OF THE INVENTION AND INFORMATION DISCLOSURE

Bacterial aggregation on the teeth known as plaque has been identified as a cause of dental caries, gingivitis, periodontitis and other gum diseases. Mechanical methods have been used for some time for the prevention of dental plaque but have not generally achieved sufficient results. Studies have shown that mechanical methods such as the use of dental floss and interspace brushes do not eliminate interproximal plaque. During the past decade or more chemical plaque control as a substitute or supplement to mechanical methods has been tried.

Octapinol has been tested for its ability to reduce plaque formation and the development of gingivitis by Willard, Edwardsson, Attstrom and Matsson, "The effect of octapinol on dento-gingival plaque and development of gingivitis," Journal of Periodontal Research, Volume 18, pages 429-437, (1983). Here it is reported that octapinol may prevent the development of plaque. Some adverse side effects of octapinol are its toxicity, lasting bitter taste and its brownish staining of the teeth.

U.S. patent no. 4,636,382 describes morpholino compounds which are useful for the inhibition or removal of dental plaque. The '382 patent also discloses that a wide variety of chemical and biological agents have been suggested for the inhibition of plaque, such as penicillin, chlorohexidine, 8-hydroxyquinoline and ethylenediamine tetraacetate. However, many of these chemical and biological agents are described as exhibiting insignificant effects and often causing serious side effects. The morpholino compounds of the '382 patent are described as having a low antibacterial effect and lacking undesirable side effects such as discoloration of the teeth.

U.S. patent No. 4,610,871 describes the use of monoalkyl and dialkyl ethers of dianhydrohexitols to inhibit the formation of plaque and calculus on teeth. U.S. patent No. 4,178,363 describes the use of n-undecylenic acid or a calcium or zinc salt thereof for reducing dental plaque and infections of the teeth and gums. U.S. patent No. 4,119,711 describes spiro 1-(hydroxyalkyl)-piperidino derivatives which have efficacy in reducing the formation of plaque. U.S. patent No. 3,976,765 describes bis-biguanido hexanes in combination with nonionic surfactants and certain foam stabilizers for use in a variety of oral preparations.

Additionally, U.S. patent No. 3,887,712 discloses that alexidine dihydrofluoride is useful in the treatment of dental plaque, calculus, gingivitis and related periodontal diseases. U.S. patent No. 4,160,821 discloses that a glycerine solution of zinc chloride or other acceptable zinc salts provides effective therapy for gingivitis when applied to the gingivae and teeth.

US-A-4 894 221 discloses compositions that inhibit the formation of dental plaque or removes plaque already formed. EP-A-0 373 758 discloses oral antiseptic compositions.

Efforts continue toward finding improved means for reducing and/or eliminating plaque without many of the side effects associated with the prior art, such as discoloration of teeth or tongue, desquamation and soreness of oral mucosa, objectionable taste, toxicity and imbalance of the oral flora. It is an object of the present invention to provide novel compositions which are useful in the treatment of plaque and gingivitis without many of the adverse side effects associated with prior art compositions. It is another object of this invention to provide anti-plaque compositions which would cause little or no ecological imbalance of the oral flora. It is a further object of this invention to provide compositions comprising a combination of a morpholinoamino alcohol and an anti-microbial agent wherein these compositions possess synergistically improved anti-plaque and anti-gingivitis activity.

### SUMMARY OF THE INVENTION

The present invention relates to novel compositions comprising a synergistic combination of a morpholinoamino alcohol or pharmaceutically-acceptable salt thereof and an anti-microbial agent. The morpholinoamino alcohol has the chemical formula 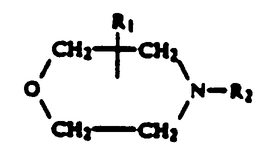
wherein R₁ is a straight or branched alkyl group containing 8 to 16 carbon atoms at the 2- or 3-position of the morpholino ring, and R₂ is a straight or branched alkyl group containing 2 to 10 carbon atoms terminating with a hydroxy group. The anti-microbial agent is selected from the group consisting of essential oils, 1-monolauroyl-rac-glycerol, 1-0-dodecyl-rac- glycerol, bis-biguanido hexane compounds, hexahydro-5-pyrimidinamine compounds, trichloro-2-hydroxy diphenyl ether compounds, quaternary ammonium compounds and pharmaceutically-acceptable salts thereof.

The preferred morpholinoamino alcohol is 3-(4-propylheptyl)-4-(2-hydroxyethyl) morpholine and its hydrochloride salt. The preferred anti-microbial agents are chlorhexidine, octenidine, cetylpyridinium chloride, hexetidine, domiphen bromide, triclosan, 1-monolauroyl-rac-glycerol, 1-0-dodecyl-rac-glycerol, thymol, eucalyptol, eugenol, methyl salicylate and mixtures thereof. The compositions preferably comprise 0.005-5% by weight of the morpholinoamino alcohol and 0.001-5.0% by weight of the anti-microbial agent. The compositions of this invention are useful in a wide variety of formulations, such as, for example, toothpaste, mouthwash, chewing gum and other product forms to reduce plaque or gingivitis. These compositions have synergistically improved anti-plaque and anti-gingivitis activity with less side effects.

### DETAILED DISCUSSION

This invention involves novel compositions which have improved anti-plaque or anti-gingivitis activity. The novel compositions of the present invention comprise a synergistic combination of a pharmaceutically-effective amount of a) one or more morpholinoamino alcohol(s) or pharmaceutically-acceptable salts thereof and b) one or more anti-microbial agent(s).

The morpholinoamino alcohols useful according to this invention have the chemical formula 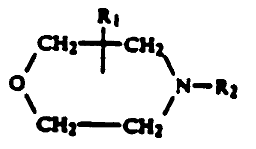
wherein R₁ is a straight or branched alkyl containing 8 to 16 carbon atoms at the 2- or 3-position of the morpholino ring, and R₂ is a straight or branched alkyl group containing 2 to 10 carbon atoms terminating with a hydroxy group. Preferably, the sum of the carbon atoms in said groups R₁ and R₂ ranges from 10 to 20, more preferably from 12 to 16. The morpholinoamino alcohols of this invention are described in U.S. patent No. 4,636,382.

The morpholinoamino alcohols can be prepared by several processes as described in U.S. patent No. 4,626,382, such as
(a) by alkylating a morpholino derivative having the formula 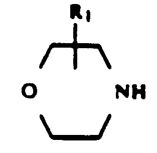 wherein R₁ is as defined above; with an alkylating agent of the formula

   R₂X

   wherein R₂ is as defined above and X is halogen or an organic sulfonic ester, or wherein X together with a hydroxyl group present in R₂ is a reactive oxide;
(b) by ring closure of a compound having the general formula 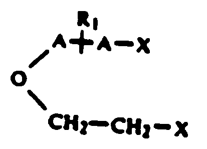 wherein R₁ is as defined above, X is halogen or an organic sulfonic ester and A represents CH₂ groups, one CH₂ group being substituted with the group R₁; with an amino alkanol of the general formula

   NH₂R₂

   wherein R₂ is as defined above;
(c) by reducing a mono- or di-oxo substituted morpholine having the general formula 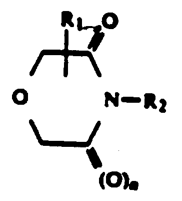 wherein R₂ is as defined above, n is 0 or 1, and R₁ is as defined above and is at the 2-position when n is 1 and at the 2- or 3-position when n is 0, or
(d) by starting from a morpholino compound having the general formula
   where R₁ is as defined above and R₃ is a straight or branched alkyl group containing a group transformable to OH or CH₂OH.

The most preferred morpholinoamino alcohol for use in this invention is 3-(4-propylheptyl)-4-(2-hydroxyethyl) morpholine represented by the chemical formula. 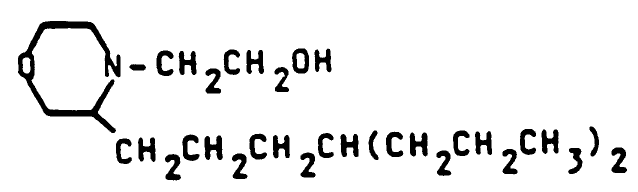
Also, most preferred is the hydrochloride salt of 3-(4-propylheptyl)-4-(2-hydroxyethyl) morpholine. This compound is a white, odorless, crystalline powder which is very soluble in water, alcohol and chloroform and has a melting point of about 70°C.

The morpholinoamino alcohols of this invention can be used in their free base form or as pharmaceutically-acceptable salts thereof. Some examples of pharmaceutically-acceptable salts are the salts of acids such as acetic acid, phosphoric acid, boric acid, hydrochloric acid, maleic acid, benzoic acid, citric acid, malic acid, oxalic acid, tartaric acid, succinic acid, glutaric acid, gentisic acid, valeric acid, gallic acid, beta- resorcylic acid, acetyl salicylic acid, salicylic acid, perchloric acid, barbituric acid, sulfanilic acid, phytic acid p-nitro benzoic acid, stearic acid, palmitic acid, oleic acid, myristic acid, lauric acid, and the like. The most preferred salts are those of hydrochloric acid.

As the morpholinoamino alcohols of this invention by themselves have only weak anti-microbial activity, it is critical to the practice of this invention that said morpholinoamino alcohols be present in combination with one or more anti-microbial agent(s). It is the synergistic combination of said morpholinoamino alcohol and an anti-microbial agent which provides the compositions of this invention with their improved anti-plaque and anti-gingivitis properties. Without being bound by any theory or mechanism of action, it is believed that these morpholinoamino alcohols inhibit key bacterial membrane functions such as carbohydrate uptake, cellular permeability, cell metabolism and cell division. Bacterial cells which are weakened by these morpholinoamino alcohols are more effectively eradicated by anti-microbial agents. Thus, compositions of this invention comprising a combination of said morpholinoamino alcohols and anti-microbial agents are extremely effective in inhibiting plaque formation and reducing preformed plaque and for treating gingivitis. These compositions have also demonstrated effectiveness in inhibiting acid production by bacteria, such as Streptococcus mutans, and therefore these compositions would have anti-caries activity.

Moreover, studies show a very low order of acute and subacute toxicity, no mutagenic activity, no adverse effect on reproduction and no staining of teeth by the morpholinoamino alcohols of the present invention.

The anti-microbial agents which are useful in combination with the morpholinoamino alcohols of this invention include essential oils, 1-monolauroyl-rac-glycerol (monolaurin), 1-0-dodecyl-rac-glycerol, bis-biguanido hexane compounds, hexahydro-5-pyrimidinamine compounds, trichloro-2-hydroxy-diphenyl ether compounds, quaternary ammonium compounds, and pharmaceutically-acceptable salts and equivalents thereof. Bis-biguanido hexane compounds are well known in the art and are described in references such as U.S. patent No. 4,241,049; 4,256,731; 4,118,474 and 3,925,543. The preferred bis-biguanido hexanes are chlorhexidine, octenidine, alexidine, and picloxydine; most preferably chlorhexidine. The quaternary ammonium compounds useful as anti-microbial agents are well known in the art and include, for example, cetylpyridinium chloride, benzethonium chloride, benzalkonium chloride, amantanium bromide, bisdequalinium chloride, cetalkonium chloride, cethexonium bromide, dequalinium acetate, dequalinium chloride, dodecarbonium chloride, domiphen bromide, laurolinium acetate, methylbenzethonium chloride, phenoctide, tibezonium iodide and triclobisonium chloride. The most preferred quaternary ammonium compound is cetylpyridinium chloride. The hexahydro-5-pyrimidamine compounds are also well known, and the preparation of these compounds is described in U.S. patent Nos. 2,415,047 and 3,054,797. Hexetidine is the most preferred hexahydro-5-pyrimidinamine compound for use in this invention. The preferred trichloro-2-hydroxy diphenyl ether compound is triclosan.

Any of the essential oils known in the art to have anti-microbial properties may be used in the present invention. The essential oils are typically volatile oil derived from the leaves, stems, flowers or twigs of plants. Chemically, they are often principally terpenes, but many other types also occur. Many essential oils are made synthetically and these synthetic essential oils are also useful in this invention. The most effective and most preferred essential oils for use in this invention are selected from the group consisting of thymol, eucalyptol, eugenol, methyl salicylate and mixtures thereof.

The above described anti-microbial agents may also be used in the form of their pharmaceutically-acceptable salts of acids such as acetic acid, phosphoric acid, boric acid, citric acid, malic acid, oxalic acid, tartaric acid, succinic acid, glutaric acid, gentisic acid, valeric acid, gallic acid, beta- resorcylic acid, acetyl salicylic acid, salicylic acid, perchloric acid, barbituric acid, sulfanilic acid, phytic acid p-nitro benzoic acid, stearic acid, palmitic acid, oleic acid, myristic acid, lauric acid and the like. The preferred salts are the digluconate, diacetate, dimonofluoro phosphate and the dihydrogen halides such as fluoride, chloride and bromide.

In the compositions of the present invention, the morpholinoamino alcohols are present preferably in an amount ranging from 0.005% to 5.0% by total weight of said composition; more preferably from 0.01% to 1.0%; and most preferably from 0.05% to 0.2%. The amount of said anti-microbial agents in the compositions preferably ranges from 0.001% to 5.0% by total weight of said compositions; more preferably from 0.002% to 2.0%; and most preferably from 0.005% to 1.0%. The specific amount of the anti-microbial agent will depend on the particular anti-microbial agent chosen and can be easily determined by those skilled in the art by routine and conventional means.

The essence of the present invention is the synergistic effect of inhibiting and reducing the growth of plaque bacteria; which is achieved when the morpholinoamino alcohols and the anti-microbial agents are utilized in combination in effective concentrations in the oral cavity. Smaller quantities of each of these components are required to obtain effective inhibition of plaque and other bacteria than if each component was utilized alone. Since lower quantities of each component can be used in the compositions of this invention, the side effects associated with each of the components would be correspondingly reduced or eliminated.

In one form of this invention the composition may be a liquid such as a mouthwash or rinse. In such a composition the vehicle is typically a water-alcohol mixture. Generally the ratio of water to alcohol is in the range of from about 1:1 to about 20:1 and most preferably 3:1 to 10:1 by weight. The most preferred mouthwash or mouthrinse compositions comprise from 0 to 30% by weight alcohol, such as ethanol. The total amount of water-alcohol mixture in a mouthwash composition is typically in the range from about 70% to about 99.9% by weight of the composition. The pH value of such mouthwash compositions is generally from about 4.0 to about 7.0 and preferably from about 4 to about 6.5. A pH below 4 would be irritating to the oral cavity. A pH greater than 7 would result in an unpleasant mouth feel.

Oral liquid and solid compositions may also contain surface active agents in amounts up to about 5% and fluorine-providing compounds in amounts up to about 2% by weight of the composition.

Surface active agents are organic materials which afford complete dispersion of the composition throughout the oral cavity. The organic surface active material may be non-ionic, amphoteric, or cationic.

Non-ionic surface active agents include condensates of sorbitan mono-oleate with from 20 to 60 moles of ethylene oxide (e.g., "Tweens" a trademark of ICI United States, Inc.), condensates of ethylene oxide with propylene oxide and condensates of propylene glycol ("Pluronics" a trademark of BASF-Wyandotte Corp.).

Other suitable non-ionic surfactants are the condensation products of an alpha-olefin oxide containing 10 to 20 carbon atoms, a polyhydric alcohol containing 2 to 10 carbons and 2 to 6 hydroxyl groups and either ethylene oxide or a heteric mixture of ethylene oxide and propylene oxide. The resultant surfactants are heteric polymers having a molecular weight in the range of about 400 to about 1600 and containing 40% to 80% by weight of ethylene oxide, with an alpha-olefin oxide to polyhydric alcohol mole ratio in the range of about 1:1 to 1:3

Amphoteric surfactants useful in the present invention are zwitterions having the capacity to act as either an acid or a base. They are generally non-irritating and non-staining. Non-limitative examples of suitable amphoteric surfactants include cocoamidopropyldimethylsultaine and cocodimethylbetaine (commercially available from Lonza Chem. Co. under the tradenames Lonzaine CS and Lonzaine 12C, respectively).

Cationic surface active agents are molecules that carry a positive charge, such as the quaternany ammonium compounds.

A fluorine providing compound may be present in the oral compositions of this invention. These compounds may be slightly water soluble or may be fully water soluble and are characterized by their ability to release fluoride ions or fluoride containing ions in water. Typical fluorine providing compounds are inorganic fluoride salts such as soluble alkali metal, alkaline earth metal, and heavy metal salts, for example, sodium fluoride, potassium fluoride, ammonium fluoride, cuprous fluoride, zinc fluoride, stannic fluoride, stannous fluoride, barium fluoride, sodium fluorosilicate, ammonium fluorosilicate, sodium fluorozirconate, sodium monofluorophosphate, aluminium mono- and difluorophosphate and fluorinated sodium calcium pyrophosphate.

In a oral liquid preparation such as a mouthwash, the fluorine providing compound is generally present in an amount sufficient to release up to about 0.15%, preferably about 0.001% to about 0.05% fluoride by weight of the preparation.

The compositions of this invention may be substantially solid or pasty in character such as dental cream, toothpaste, toothpowder, or chewing gum. Solid or pasty oral compositions contain polishing materials. Typical polishing materials are abrasive particulate materials having particle sizes of up to about 20 »m. Nonlimiting illustrative examples include: water-insoluble sodium metaphosphate, potassium metaphosphate, tricalcium phosphate, dehydrated calcium phosphate, anhydrous dicalcium phosphate, calcium pyrophosphate, magnesium orthophosphate, trimagnesium phosphate, calcium carbonate, alumina, aluminum silicate, zirconium silicates, silica, bentonite, and mixtures thereof. Polishing materials are generally present in an amount from about 20% to about 75% in toothpaste, and from about 70% to about 99% in toothpowder.

In clear gels, a polishing agent of colloidal silica and alkali metal aluminosilicate complexes are preferred since they have refractive indicies close to the refractive indicies of gelling agent liquid systems commonly used in dentifrices.

The compositions of the present invention may additionally contain sweeteners, flavorants and colorants.

In the instance where auxiliary sweeteners are utilized, the present invention contemplates the inclusion of those sweeteners well known in the art, including both natural and artificial sweeteners. Thus, additional sweeteners may be chosen from the following non-limiting list:
A. Water-soluble sweetening agents such as monosaccharides, disaccharides and polysaccharides such as xylose, ribose, glucose, mannose, galactose, fructose, dextrose, sucrose, maltose, partially hydrolyzed starch, or corn syrup solids and sugar alcohols such as sorbitol, xylitol, mannitol and mixtures thereof.
B. Water-soluble artificial sweeteners such as the soluble saccharin salts, i.e., sodium, or calcium saccharin salts, cyclamate salts, acesulfame-K and the like, and the free acid form of saccharin.
C. Dipeptide based sweeteners such as L-phenylalanine methyl ester and materials described in U.S. Patent No. 3,492,131 and the like.

In general, the amount of sweetener will vary with the desired amount of sweetness selected for a particular composition. This amount will normally be 0.01% to about 40% by weight. The water-soluble sweeteners described in category A above, are preferably used in amounts of about 5% to about 40% by weight, and most preferably from about 10% to about 20% by weight of the final composition. In contrast, the artificial sweeteners described in categories B and C are used in amounts of about 0.005% to about 5.0% and most preferably about 0.05% to about 2.5% by weight of the final composition. These amounts are ordinarily necessary to achieve a desired level of sweetness independent from the flavor level achieved from flavorants.

Suitable flavorings include both natural and artificial flavors, and mints such as peppermint, citrus flavors such as orange and lemon, artificial vanilla, cinnamon and various fruit flavors and the like. Both individual and mixed flavors are comtemplated. The flavorings are generally utilized in amounts that will vary depending upon the individual flavor, and may, for example, range in amounts of about 0.1% to about 6% by weight of the final composition.

The colorants useful in the present invention, include the pigments which may be incorporated in amounts of up to about 2% by weight of the composition. Also, the colorants may include other dyes suitable for food, drug and cosmetic applications, and known as F.D. & C. dyes and the like. The materials acceptable for the foregoing spectrum of use are preferably water-soluble. Illustrative examples include the indigo dye, known as F.D. & C. Blue No. 2, which is the disodium salt of 5,5-indigotindisulfonic acid. Similarly, the dye known as F.D. & C. Green No. 1, comprises a triphenylmethane dye and is the monosodium salt of 4-[4-N-ethyl-p-sulfo-benzylamino)dephenylmethylene]-[1-(N-ethyl-N-p-sulfonium-benzyl)-2,5-cyclo-hexadienimine]. A full recitation of all F.D. & C. and D. & C. colorants useful in the present invention and their corresponding chemical structures may be found in the Kirk-Othmer Encyclopedia of Chemical Technology, 3rd Edition, in Volume 6, at pages 561-595.

The present invention is also related to a method for treating teeth and gums to reduce plaque or gingivitis comprising applying to the surface of the teeth and/or gums the compositions of this invention as described earlier. The compositions can be applied to the teeth and gums by any conventional means such as brushing, spraying, painting or rinsing of the oral cavity and the like. The compositions not only retard plaque accumulation, but are effective in removing pre-existing plaque as well. Additionally, the compositions show a prolonged effect on plaque accumulation following cessation of treatment through about one week after use. The compositions of this invention are also useful as a topical antiseptic or disinfectant which is applied externally to the skin.

The following examples are presented to further illustrate this invention. The examples are intended in an illustrative sense and not in a limitative sense. This invention includes the embodiments described herein and equivalents thereof. All parts and percentages used herein are on a weight basis unless otherwise indicated.

### Example I

A composition within the scope of this invention was prepared by mixing the ingredients presented in Table I below. This composition was useful as a mouthwash.

**Table I**

| Ingredient | Amount (Percent w/v) |
|---|---|
| 3-(4-propylheptyl)-4-(2-hydroxyethyl) morpholine hydrocholoride | 0.05 |
| 1-monolauroyl-rac-glycerol | 0.05 |
| non-ionic surfactant | 0.7 |
| sorbitol solution (70% solids) | 50.0 |
| ethanol (95% in water) | 10.0 |
| coloring agent | 0.0004 |
| flavoring agent | 0.15 |
| deionized water | (quantity sufficient to 100%) |

### Example II

A composition within the scope of this invention was prepared by mixing the ingredients presented in Table II below. This composition was useful as a mouthwash.

**Table II**

| Ingredient | Amount (Percent w/v) |
|---|---|
| 3-(4-propylheptyl)-4-(2-hydroxyethyl) morpholine hydrochloride | 0.05 |
| thymol | 0.6 |
| eucalyptol | 0.6 |
| menthol | 0.6 |
| methyl salicylate | 0.6 |
| non-ionic surfactant | 1.0 |
| sorbitol solution (70% solids) | 50.0 |
| ethanol (95% in water) | 25.0 |
| coloring agent | 0.005 |
| flavoring agent | 0.15 |
| deionized water | (quantity sufficient to 100%) |

### Example III

A composition within the scope of this invention was prepared by mixing the ingredients presented in Table III below. This composition was useful as an oral spray.

**Table III**

| Ingredient | Amount (Percent w/v) |
|---|---|
| 3-(4-propylheptyl)-4-(2-hydroxyethyl) morpholine hydrochloride | 0.10 |
| hexetidine | 0.10 |
| non-ionic surfactant | 1.2 |
| citric acid; hydrous | 0.07 |
| ethanol (95% in water) | 12.0 |
| glycerol | 20.0 |
| sweetening agent | 0.01 |
| flavoring agent | 0.10 |
| deionized water | (quantity sufficient to 100%) |

### Example IV

Compositions within the scope of the present invention were prepared by mixing the ingredients presented in Table IV below. These compositions were useful as a dentrifice.

**Table IV**

| Ingredient | Amount (Percent w/v) |
|---|---|
| 3-(4-propylheptyl)-4-(2-hydroxyethyl) morpholine hydrochloride | 0.2 |
| Triclosan | 0.3 |
| sodium fluoride | 0.24 |
| hydrated silica | 10 - 50 |
| xylitol | 10 - 40 |
| xanthan gum | 0.1 - 1.5 |
| cocobetaine | 0.1 - 1.5 |
| flavor | 0.9 |
| deionized water | (quantity sufficient to 100%) |

### Example V

A gel composition within the scope of this invention was prepared by mixing the ingredients presented in Table V below.

**Table V**

| Ingredient | Amount (Percent w/v) |
|---|---|
| 3-(4-propylheptyl)-4-(2-hydroxyethyl) morpholine hydrochloride | 0.3 |
| chlorhexidine gluconate | 0.2 |
| methyl cellulose | 2.0 |
| sorbitol solution (70% solids) | 50.0 |
| flavoring agent | 0.2 |
| deionized water | (quantity sufficient to 100%) |

### Example VI

A composition within the scope of this invention was prepared by mixing the ingredients presented in Table VI below. This composition was useful as a mouthwash.

**Table VI**

| Ingredient | Amount (Percent w/v) |
|---|---|
| 3-(4-propylheptyl)-4-(2-hydroxyethyl) morpholine hydrochloride | 0.05 |
| cetyl pyridinium chloride | 0.002 |
| non-ionic surfactant | 0.7 |
| sorbitol solution (70% solids in water) | 50.0 |
| ethanol | 10.0 |
| coloring agent | 0.004 |
| flavoring agent | 0.15 |
| deionized water | (quantity sufficient to 100%) |

### Example VII

### MINIMAL GROWTH INHIBITORY CONCENTRATION

The minimal inhibitory concentration (MIC) of the test compound is the minimum concentration of compound required to inhibit growth of the test microorganism. The MIC is determined by serially diluting the test solution through tubes of sterile broth. The tubes are inoculated and incubated at 37°C for 48 hours. At the end of the incubation time, growth is checked macroscopically. The lowest concentration of test compound to inhibit growth is the MIC.

### CHECKERBOARD TITRATION

This technique is used to assess antimicrobial activity of combinations. The checkerboard is the pattern found by multiple combinations of two agents in concentrations equal to, above and below their minimal inhibitory concentration for the microorganism tested against. The checkerboard consists of columns which contain the same amount of one agent which is diluted along the x-axis and rows which each contain the same amount of the second agent diluted along the y-axis. Thus each tube is a unique combination of the two agents. It is possible that one or both of the agents alone possess no anti-microbial activity while combinations at the appropriate concentrations demonstrate effective antimicrobial activity.

A method for illustrating the effect of the combination of the two agents is obtained by plotting the MIC's of each agent alone and the MIC's for these agents in combination. Antimicrobial synergy is indicated if the MIC's for the agents in combination are lower than the MIC's for the agents alone.

The results presented in Table VII below demonstrate the synergistic combination of 3-(4-propylheptyl)-4-(2-hydroxyethyl) morpholine hydrochloride and the anti-microbial 1-0-dodecyl-rac-glycerol against two representative oral bacteria (Streptococcus mutans, a gram-positive coccus and ediological agent of dental caries, and Bacteroides melaninogenicus, a gram-negative obligate anearobe associated with gingivitis and periodontal disease).

**Table VII**

| Organism | Minimum Inhibitory Concentration 3-(4-propylheptyl)-4-(2-hydroxyethyl) morpholine hydrochloride | (miligrams/liter) 1-0-dodecyl-rac-glycerol |
|---|---|---|
| S. mutans | 200 | 0 |
| | 100 | 2 |
| | 4 | 6 |
| | 0 | 10 |
| B. melaninogenicus | 200 | 0 |
| | 20 | 2 |
| | 4 | 6 |
| | 0 | 10 |

The above data shows that, in the absence of the dodecyl glycerol, the minimum inhibitory concentration of the morpholinoamino alcohol HC1 for both test microorganisms was 200 milligrams per liter. Increasing the concentration of the dodecyl glycerol synergistically decreased the minimum inhibitory concentration of the morpholinoamino alcohol HC1. Likewise, increasing the concentration of the morpholinoamino alcohol HC1 synergistically decreased the minimum inhibitory concentration of the dodecyl glycerol. These results are indicative of anti-microbial synergy as described by Krogstad and Moellerging, Antibiotics in Laboratory Medicine, V. Lorian (editor), Williams & Wilkins, p. 302, (1980).

### Example VIII

Another example of synergistic combinations is presented in Table VIII below. The results in Table VIII demonstrate the synergistic combination of 3-(4-propylhepty)-4-(2-hydroxyethyl) morpholine hydrochloride and the antimicrobial agent cetyl pyridinium chloride against S. mutans (ATCC 2517S) and B. melaninogenicus (J18M2). By increasing the concentration of cetyl pyridinium chloride, the minimum inhibitory concentration of the morpholinoamino alcohol HCl is synergistically decreased. Likewise, increasing the concentration of the morpholinoamino alcohol HCl synergistically decreased the minimum inhibitory concentration of cetyl pyridinium chloride.

**Table VIII**

| Organism | Minimum Inhibitory Concentration 3-(4-propylheptyl)-4-(2-hydroxyethyl) morpholine hydrochloride | (milligrams/liter) cetyl pyridinium chloride |
|---|---|---|
| S. mutans | 200 | 0 |
| | 100 | 0.0125 |
| | 50 | 0.0188 |
| | 0 | 0.025 |
| B. melaninogenicus | 200 | 0 |
| | 100 | 0.003 |
| | 50 | 0.006 |
| | 0 | 0.0125 |

### Example IX

A further example of synergistic combinations is presented in Table IX below. The results in Table IX demonstrate the synergistic combination of 3-(4-propylheptyl)-4 -(2-hydroxyethyl) morpholine hydrochloride and the anti-microbial agent domiphen bromide against s. mutans and B. melaninogenicus.

**Table IX**

| Organism | Minimum Inhibitory Concentration 3-(4-propylheptyl)-4-(2-hydroxyethyl) morpholine hydrochloride | (milligrams/liter) domiphen bromide |
|---|---|---|
| S. mutans | 200 | 0 |
| | 100 | 0.5 |
| | 50 | 1.0 |
| | 0 | 1.0 |
| B. melaninogenicus | 200 | 0 |
| | 100 | 6.25 |
| | 50 | 12.5 |
| | 0 | 50 |

### Example X

A further example of synergistic combinations is presented in Table X below. The results in Table X demonstrate the synergistic combination of 3-(4-propylheptyl)-4 -(2-hydroxyethyl) morpholine hyerochloride and the antimicrobial agent hexetidine against s. mutans and B. melaninogenicus.

**Table X**

| Organism | Minimum Inhibitory Concentration 3-(4-propylheptyl)-4-(2-hydroxyethyl) morpholine hydrochloride | (milligrams/liter) hexitidine |
|---|---|---|
| S. mutans | 200 | 0 |
| | 100 | 0.25 |
| | 50 | 0.25 |
| | 0 | 1.0 |
| B. melaninogenicus | 200 | 0 |
| | 100 | 0.125 |
| | 50 | 0.25 |
| | 0 | 1.0 |

### Example XI

Following the procedures of Examples VII-X, combinations of 3-(4-propylheptyl)-4-(2-hydroxyethyl) morpholine hydrochloride with amyl-m-cresol and 2,4-dichlorobenzyl alcohol, respectively, were prepared for comparative purposes. These combinations were evaluated for antimicrobial and synergistic activity against s. mutans and B. melaninogenicus. The results of these evaluation are presented in Tables XI and XII and do not show any relative synergistic anti-microbial activity from the combinations.

**Table XI**

| Organism | Minimum Inhibitory Concentration 3-(4-propylheptyl)-4-(2-hydroxyethyl) morpholine hydrochloride | (milligrams/liter) amyl-m-cresol |
|---|---|---|
| S. mutans | 200 | 0 |
| | 100 | 10 |
| | 50 | 10 |
| | 0 | 10 |
| B. melaninogenicus | 200 | 0 |
| | 100 | 2.5 |
| | 50 | 10 |
| | 0 | 10 |

**Table XI**

| Organism | Minimum Inhibitory Concentration 3-(4-propylheptyl)-4-(2-hydroxyethyl) morpholine hydrochloride | (milligrams/liter) 2, 4-dichloro-alcohol |
|---|---|---|
| S. mutans | 200 | 0 |
| | 100 | 50 |
| | 50 | 50 |
| | 0 | 50 |
| B. melaninogenicus | 200 | 0 |
| | 100 | 100 |
| | 50 | 100 |
| | 0 | 100 |

## Claims

1. A composition having anti-plaque or anti-gingivitis activity comprising in combination a pharmaceutically effective amount of a) a morpholinoamino alcohol or pharmaceutically-acceptable salt thereof, wherein said morpholinoamino alcohol has the chemical formula 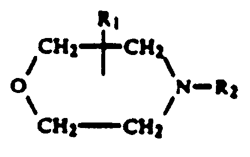 wherein R₁ is a straight or branched alkyl group containing 8 to 16 carbon atoms at the 2- or 3-position of the morpholino ring, and R₂ is a straight or branched alkyl group containing 2 to 10 carbon atoms terminating with a hydroxy group and b) an anti-microbial agent selected from the group consisting of 1-monolauroyl-rac -glycerol, 1-0-dodecyl-rac-glycerol, thymol, eucalyptol, eugenol, bis-biguanido hexane compounds, hexahydro-5-pyrimidinamine compounds, trichloro-2-hydroxy-diphenyl ether compounds, quaternary ammonium compounds, methyl salicylate and pharmaceutically-acceptable salts thereof.

2. The composition of claim 1 wherein said anti-microbial agent is selected from the group consisting of 1-monolauroyl-rac-glycerol, 1-0-dodecyl-rac-glycerol, chlorhexidine, octenidine, cetylpyridinium chloride, hexetidine, domiphen bromide and triclosan.

3. The composition of claim 1 wherein the sum of the carbon atoms in said groups R₁ and R₂ is 10 to 20.

4. The composition of claim 1 wherein said morpholinoamino alcohol is 3-(4-propylheptyl)-4-(2-hydroxyethyl) morpholine or its hydrochloride salt.

5. The composition of claim 1 wherein the amount of said morpholinoamino alcohol ranges from 0.005% to 5.0% by weight of said composition.

6. The composition of claim 1 wherein the amount of said morpholinoamino alcohol ranges from 0.01% to 1.0% by weight of said composition.

7. The composition of claim 1 wherein the amount of said anti-microbial agent ranges from 0.001% to 5.0% by weight of said composition.

8. The composition of claim 1 wherein the amount of said anti-microbial agent ranges from 0.002% to 2.0% by weight of said composition.

9. The composition of claim 1 wherein said pharmaceutically-acceptable salts are the salts of acids selected from the group consisting of acetic acid, phosphoric acid, boric acid, hydrochloric acid, maleic acid, benzoic acid, citric acid, malic acid, oxalic acid, tartaric acid, succinic acid, glutaric acid, gentisic acid, valeric acid, gallic acid, beta- resorcylic acid, acetyl salicylic acid, salicylic acid, perchloric acid, barbituric acid, sulfanilic acid, phytic acid, p-nitro benzoic acid, stearic acid, palmitic acid, oleic acid, myristic acid and lauric acid.

10. The composition of claim 1 wherein said composition is a tooth paste.

11. The composition of claim 1 wherein said composition is a liquid mouthwash.

12. The composition of claim 1 wherein said composition is a chewing gum.

13. The composition of claim 11 wherein said mouthwash comprises from 0 to 30% by weight alcohol.

14. The composition of claim 13 wherein said alcohol is ethanol.

15. Use of compositions according to claims 1 to 9 for the production of pharmacologically effective preprarations for the treatment of diseases of the teeth and gums, for the inhibition of dental plaque growth and removal of dental plaque.

16. Process for the production of preparations according to claims 10 to 14, characterized in that the components a) and b) of claim 1 are incorporated with suitable adjuvants and excipients in tooth pastes, mouthwashes, mouthrinses, oral sprays, dentifrices, oral gels or chewing gums.

## Patentansprüche

1. Zusammensetzung mit einer Anti-Plaque- oder Anti-Gingivitis-Aktivität, die in Kombination enthält (umfaßt) eine pharmazeutisch wirksame Menge von
a) einem Morpholinoaminoalkohol oder einem pharmazeutisch akzeptablen Salz desselben, wobei der Morpholinoaminoalkohol die chemische Formel hat 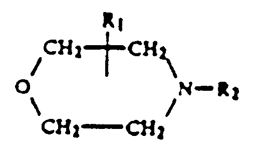 worin bedeuten:
R₁ eine gerade (unverzeigte) oder verzweigte Alkylgruppe mit 8 bis 16 Kohlenstoffatomen in der 2- oder 3-Position des Morpholinringes und
R₂ eine gerade (unverzeigte) oder verzweigte Alkylgruppe mit 2 bis 10 Kohlenstoffatomen, die mit einer Hydroxygruppe endet, und
b) einem antimikrobiellen Agens, ausgewählt aus der Gruppe, die besteht aus 1-Monolauroyl-rac-glycerin, 1-O-Dodecyl-rac-glycerin, Thymol, Eucalyptol, Eugenol, Bis-biguanido-hexan-Verbindungen, Hexahydro-5-pyrimidinamin-Verbindungen, Trichloro-2-hydroxy-diphenyläther-Verbindungen, quaternären Ammoniumverbindungen, Methylsalicylat und pharmazeutisch akzeptablen Salzen davon.

2. Zusammensetzung nach Anspruch 1, worin das antimikrobielle Agens ausgewählt wird aus der Gruppe, die besteht aus 1-Monolauroyl-rac-glycerin, 1-O-Dodecyl-rac-glycerin, Chlorhexidin, Octenidin, Cetylpyridiniumchlorid, Hexetidin, Domiphenbromid und Triclosan.

3. Zusammensetzung nach Anspruch 1, worin die Summe der Kohlenstoffatome in den genannten Gruppen R₁ und R₂ 10 bis 20 beträgt.

4. Zusammensetzung nach Anspruch 1, worin der genannte Morpholinoaminoalkohol 3-(4-Propylheptyl)-4-(2-hydroxyethyl)morpholin oder sein Hydrochloridsalz ist.

5. Zusammensetzung nach Anspruch 1, worin die Menge des genannten Morpholinoaminoalkohols in dem Bereich von 0,005 bis 5,0 Gew.-%, bezogen auf die Zusammensetzung, liegt.

6. Zusammensetzung nach Anspruch 1, worin die Menge des genannten Morpholinoaminoalkohols in dem Bereich von 0,01 bis 1,0 Gew.-%, bezogen auf die Zusammensetzung, liegt.

7. Zusammensetzung nach Anspruch 1, worin die Menge des genannten antimikrobiellen Agens in dem Bereich von 0,001 bis 5,0 Gew.-%, bezogen auf die Zusammensetzung, liegt.

8. Zusammensetzung nach Anspruch 1, worin die Menge des genannten antimikrobiellen Agens in dem Bereich von 0,002 bis 2,0 Gew.-%, bezogen auf die Zusammensetzung, liegt.

9. Zusammensetzung nach Anspruch 1, worin die genannten pharmazeutisch akzeptablen Salze die Salze von Säuren sind, die ausgewählt werden aus der Gruppe, die besteht aus Essigsäure, Phosphorsäure, Borsäure, Chlorwasserstoffsäure, Maleinsäure, Benzoesäure, Citronensäure, Apfelsäure, Oxalsäure, Weinsäure, Bernsteinsäure, Glutarsäure, Gentisinsäure, Valeriansäure, Gallussäure, β-Resorcylsäure, Acetylsalicylsäure, Salicylsäure, Perchlorsäure, Barbitursäure, Sulfanilsäure, Phytinsäure, p-Nitrobenzoesäure, Stearinsäure, Palmitinsäure, Ölsäure, Myristinsäure und Laurinsäure.

10. Zusammensetzung nach Anspruch 1, worin die genannte Zusammensetzung eine Zahnpasta ist.

11. Zusammensetzung nach Anspruch 1, worin die genannte Zusammensetzung ein flüssiges Mundwasser ist.

12. Zusammensetzung nach Anspruch 1, worin die genannte Zusammensetzung ein Kaugummi ist.

13. Zusammensetzung nach Anspruch 11, worin das genannte Mundwasser 0 bis 30 Gew.-% Alkohol enthält.

14. Zusammensetzung nach Anspruch 13, worin der genannte Alkohol Ethanol ist.

15. Verwendung der Zusammensetzungen nach den Ansprüchen 1 bis 9 für die Herstellung von pharmakologisch wirksamen Präparaten zur Behandlung von Erkrankungen der Zähne und des Zahnfleisches, zur Inhibierung (Hemmung) des Wachstums der Zahn-Plaque (Zahnbelags) und zur Entfernung der Zahn-Plaque (des Zahnbelags).

16. Verfahren zur Herstellung von Präparaten nach den Ansprüchen 10 bis 14, dadurch gekennzeichnet, daß die Komponenten (a) und (b) des Anspruchs 1 zusammen mit geeigneten Adjuvantien und Exzipienten Zahnpasten, Mundwässern, Mundduschen, Oralsprays, Zahncremes, Oralgelen oder Kaugummis einverleibt werden.

## Revendications

1. Combinaison présentant une activité anti-plaque dentaire ou anti-gingivite comprenant en combinaison une quantité efficace pharmaceutiquement de a) un alcool morpholinoamino ou son sel pharmaceutiquement acceptable, dans laquelle l'alcool morpholinoamino a la formule chimique 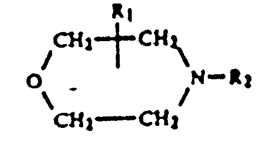 dans laquelle R₁ est un groupe alkyle linéaire ou ramifié contenant 8 à 16 atomes de carbone en position 2 ou 3 du cycle morpholino, et R₂ est un groupe alkyle linéaire ou ramifié contenant 2 à 10 atomes de carbone se terminant par un groupe hydroxy et b) un agent anti-microbien choisi dans le groupe constitué par 1-monolauroyl-rac-glycérol, 1-0-dodécyl-rac-glycérol, des composés bis-biguanido hexane, des composés hexahydro-5-pyrimidinamine, des composés de trichloro-2-hydroxy diphényléther, des composés d'ammonium quaternaire, du méthylsalicylate et leurs sels acceptables pharmaceutiquement.

2. Composition selon la revendication 1, dans laquelle l'agent anti-microbien est choisi dans le groupe constitué par 1-monolauroyl-rac-glycérol, 1-0-dodécyl-rac-glycérol, chlorohexidine, octénidine, chlorure de cétylpyridinium, hexetidine, bromure de domiphène et triclosan.

3. Composition selon la revendication 1, dans laquelle la somme des atomes de carbone dans les groupes R₁ et R₂ est égale à 10 jusqu'à 20.

4. Composition selon la revendication 1, dans laquelle l'alcool morpholinoamino est la morpholine 3-(4-propylheptyl)-4-(2-hydroxyéthyl) ou son sel de chlorhydrate.

5. Composition selon la revendication 1, dans laquelle la quantité de l'alcool morpholinoamino varie de 0,005 % jusqu'à 5,0 % en poids de la composition.

6. Composition selon la revendication 1, dans laquelle la quantité d'alcool morpholinoamino varie de 0,01 % jusqu'à 1,0 % en poids de la composition.

7. Composition selon la revendication 1, dans laquelle la quantité de l'agent anti-microbien varie de 0,001 % jusqu'à 5,0 % en poids de la composition.

8. Composition selon la revendication 1, dans laquelle la quantité de l'agent anti-microbien varie de 0,002 % jusqu'à 2,0 % en poids de la composition.

9. Composition selon la revendication 1, dans laquelle les sels acceptables pharmaceutiquement sont les sels des acides choisis dans le groupe constitué par l'acide acétique, l'acide phosphorique, l'acide borique, l'acide chlorhydrique, l'acide maléique, l'acide benzoïque, l'acide citrique, l'acide malique, l'acide oxalique, l'acide tartarique, l'acide succinique, l'acide glutarique, l'acide gentisique, l'acide valérique, l'acide gallique, l'acide bêta-résorcylique, l'acide acétylsalicylique, l'acide salicylique, l'acide perchlorique, l'acide barbiturique, l'acide sulfanilique, l'acide phytique, l'acide p-nitro benzoïque, l'acide stéarique, l'acide palmitique, l'acide oléique, l'acide myristique et l'acide laurique.

10. Composition selon la revendication 1, dans laquelle la composition est une pâte dentaire.

11. Composition selon la revendication 1, dans laquelle la composition est un collutoire liquide.

12. Composition selon la revendication 1, dans laquelle la composition est une gomme à mâcher.

13. Composition selon la revendication 11, dans laquelle l'élixir dentaire comprend de 0 à 30 % en poids d'alcool.

14. Composition selon la revendication 13, dans laquelle l'alcool est l'éthanol.

15. Utilisation des compositions selon les revendications 1 à 9 pour la production de préparations efficaces pharmacologiquement pour le traitement des affections dentaires et gingivales, pour l'inhibition de la formation de plaque dentaire et pour la suppression de la plaque dentaire.

16. Procédé pour la production de préparations selon les revendications 10 à 14, caractérisé en ce que les composants a) et b) de la revendication 1 incorporent des adjuvants appropriés et des excipients dans les pâtes dentaires, les collutoires, les gargarismes, les sprays buccaux, les dentifrices, les gels buccaux ou les gommes à mâcher.
